# EUROPEAN PATENT SPECIFICATION

(11) **EP 4 366 798 B1**
(45) Date of publication and mention of the grant of the patent: **25.02.2026**
(21) Application number: 22828905.4
(22) Date of filing: 20.06.2022
(51) Int. Cl.: A61M 5/20, A61M 5/31, A61M 5/315, A61M 5/32

(54) **MAGNETIC AUTOINJECTOR**
MAGNETISCHER AUTOINJEKTOR
AUTO-INJECTEUR MAGNÉTIQUE

(30) Priority: 22.06.2021 TR 202110143
(43) Date of publication of application: 15.05.2024
(73) Proprietor: Argeron Medikal Arastirma Sanayi Ve Ticaret Anonim Sirketi, Antalya (TR)
(72) Inventor: BIDI, Bülent, 7160 Antalya (TR); OLGUN, Abdullah, Antalya (TR)
(74) Representative: Yamankaradeniz, Kemal
(86) International application number: PCT/TR2022/050621
(87) International publication number: WO 2022/271133

(56) References cited:
- WO-A1-2014/162439
- CN-Y- 201 044 845
- US-A1- 2011 077 599
- US-A1- 2013 310 745
- US-A1- 2018 193 564
- US-A1- 2019 022 318
- US-A1- 2020 107 764

## Description

### TECHNICAL FIELD

The invention generally relates to injectors (syringes) used for injecting drugs into the living body.

In particular, the invention requires first aid and emergency intervention to oneself or others; It is about the magnetic autoinjector, which is used in cases where intradermal, subcutaneous or intramuscular injection is needed in the living body and provides a safe and easy use.

### BACKGROUND OF THE ART

Today, injectors are used in cases where it is necessary to inject drugs intrdermally, subcutaneously or intramuscularly in the living body. In the most general form, injectors consist of a tube in which the drug to be injected is filled, a needle that allows the drug to be transferred to the living body, and a pushing element that allows the drug to be pushed.

In the current art, injectors have been developed in different designs and working styles according to the needs. One of these injector types is autoinjectors. Autoinjectors are designed to deliver a dose of a particular drug. There are two groups of autoinjectors, with and without needles. The disadvantage of the ones with needle is that they have complex working principles with a standard long injector, a piston rod and a spring. The biggest disadvantage of needleless ones (jet injectors) is that they need a non-disposable special apparatus for application and the risk of infection.

In the state of the art, some documents were found in the research on injectors. The document numbered WO2014162439A1 is about a syringe type injector without a piston, which is easy to carry and aims to inject the drug solution in a simple manner. In this syringe, a pushing structure is formed in its body, which uses the magnetic force to discharge the drug solution. In this structure, a magnet is placed in a fixed holder at the back of the body and another magnet is placed in the inner holder that allows the drug to be injected. Said magnets are positioned inside the fixed holder and the inner holder in such a way that the same poles are facing each other, that is, they are positioned in such a way that the fixed holder and the inner holder repel each other. At the same time, the inner holder is locked by a stopper outside the body. The inner holder, which is released after the movement of the stopper, moves towards the end of the body as a result of the magnets pushing each other, and the drug solution is provided to come out of the body end. In this embodiment, the magnet only provides the discharge of the liquid in the syringe, while the entry of the syringe needle into the skin is provided by the user. At the same time, said injector needle is outside the injector body and is fixed. With this structure, the injector configuration mentioned does not provide a fast, easy and safe use.

Document EP3338833B1 is about an automatic injector. In this document, a pushing mechanism with a spring mechanism is used to throw the liquid drug substance found in the syringe out of the syringe. The drive of this spring mechanism is provided by a mechanical trigger button placed outside the injector. In this embodiment, the piston movement of the injector and the insertion of the needle into the skin are provided by a spring mechanism. The size of the spring mechanism used affects the size of the injector body. In addition, the needle coming out of the injector body remains at the outside. In this state, the mentioned structure cannot go below a certain size, and leaving the needle out is a disadvantage in terms of safety.

Document US 2019/022318 A1 is about an injection device. The injection device uses magnets and magnetic force for driving an injection pin distally to insert a needle and push a plunger to expel a liquid out of a cartridge. The injection device has a housing that houses the driver magnets and further has the injection pen that is coupled to the actuator magnets. In a pre-injection position there is attracting force between the driver magnets and the actuator magnets, once the injection device is pushed against a skin of a user, the relative position between the driver magnets and the actuator magnets changes which leads into a repulsive force which drives the injection pin distally.

As a result, improvements are made regarding injectors, therefore, new structures are needed that will eliminate the disadvantages mentioned above and bring solutions to existing systems.

### OBJECTIVE OF THE INVENTION

The present invention relates to a magnetic autoinjector that meets the above-mentioned requirements, eliminates all disadvantages and brings some additional advantages.

The main purpose of the invention is to ensure that a certain dose of the drug is automatically injected intradermally, subcutaneously or intramuscularly in the living body.

An aim of the invention is to ensure that the injection process is carried out automatically with a structure that does not need a piston rod and a spring mechanism. By not using the piston rod and the spring mechanism, it is aimed to introduce an injector that is shorter and smaller than the existing technique.

In order to realize all the advantages mentioned above and which will be understood from the detailed description below, the present invention is a magnetic autoinjector that is used in cases where intradermal, subcutaneous or intramuscular injection is needed in the living body, allowing a certain dose of liquid medication to be injected into the living body easily and safely; so that having a structure containing:
- the injector body, which is in a closed cylindrical form and having an outlet opening at the end,
- stationary magnet, which is positioned in the injector body, mostly in the middle of the injector body, with an inlet opening on it,
- a needle magnet with a needle on it, positioned in the injector body, between the stationary magnet and the outlet opening, close to the stationary magnet and with the same poles facing each other in order to ensure that they apply a repulsive force,
- a piston magnet positioned in the injector body, on the other side of the stationary magnet, with the opposite poles facing each other and with liquid medication between them, in order to ensure that they apply a pulling force to each other,
- The transfer channel located inside the stationary magnet and the needle magnet, which provides the transfer of the liquid drug entering the inlet opening to the needle,
- locking elements that keep the piston magnet and needle magnet stationary against pulling and pushing forces when the injector is not used.

The structural and characteristic features of the invention and all its advantages will be understood more clearly thanks to the figures given below and the detailed description written with reference to these figures. For this reason, the evaluation should be made by taking these figures and detailed explanation into consideration.

### BRIEF DESCRIPTION OF THE FIGURES

In order to best understand the embodiment of the present invention and its advantages with additional elements, it should be evaluated together with the figures described below.

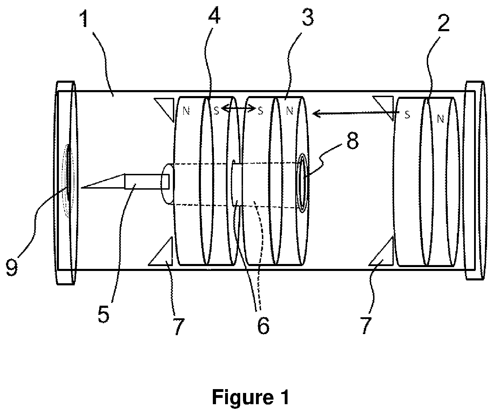

Figure 1 is a general view of the magnetic autoinjector which is the subject of the invention.

### REFERENCE NUMBERS

1. Injector body
2. Piston magnet
3. Stationary magnet
4. Needle magnet
5. Needle
6. Transfer channel
7. Locking element
8. Inlet opening
9. Exit opening

### DETAILED DESCRIPTION OF THE INVENTION

In this detailed explanation, the preferred embodiments of the magnetic autoinjector subject to the invention are explained only for a better understanding of the subject and without any limiting effect.

The invention is a magnetic autoinjector, which is used in cases where intradermal, subcutaneous or intramuscular injection is needed in the living body, allowing a certain dose of liquid medicine to be injected into the living body easily and safely; that contains;
- the injector body (1), which has a closed cylindrical form and has an outlet opening (9) at the end,
- a stationary magnet (3) with an inlet opening (8) positioned in a stationary manner in the injector body (1), mostly in the middle region of the injector body (1),
- a needle magnet (4) with a needle (5) on it, positioned in the injector body (1), between the stationary magnet (3) and the outlet opening (9), close to the stationary magnet (3) and with the same poles facing each other to enable them to apply a repulsive force.
- a piston magnet (2) located inside the injector body (1), on the other side of the stationary magnet (3), with its opposite poles facing each other and with liquid medicine between them, in order to provide a pulling force to each other,
- the transfer channel (6), located inside the stationary magnet (3) and the needle magnet (4), which provides the transfer of the liquid medicine entering the inlet opening (8) to the needle (5),
- locking elements (7) that keep the piston magnet (2) and needle magnet (4) in a stationary manner against pulling and pushing forces when the injector is not in use.

In Figure 1, there is a general view of the magnetic injector which is the subject of the invention. Said magnetic autoinjector in its most general form consists of an injector body (1), a piston magnet (2) positioned in it, a stationary magnet (3) and a needle magnet (3) to which the needle (5) is connected. The injector body (1) has a cylindrical structure similar to the existing injectors and includes an exit opening (9) at its tip that allows the needle (5) to come out. Piston magnet (2), stationary magnet (3) and needle magnet (4) are positioned respectively on the inside of the injector body (1), again in accordance with the form of the injector body (1). The piston magnet (2) provides pushing the liquid medicine found inside the injector body (1) and between the piston magnet (2) and the stationary magnet (3). The needle magnet (4) carries the needle (5) on it and provides its movement. The stationary magnet (2) is located in the middle of the injector body (1) and provides the movement of the piston magnet (2) and the needle magnet (4). In order to achieve this, the opposite poles of the piston magnet (2) and the stationary magnet (3) face each other, and the same poles of the needle magnet (4) and the stationary magnet (3) face each other. In this state, the stationary magnet (3), while pulling the piston magnet (2) towards itself, pushes the needle magnet (4) and causes the needle (5) to come out of the exit opening (9). At the same time, there is a transfer channel (6) that passes through the stationary magnet (3) and the needle magnet (4) in order to ensure that the liquid drug between the piston magnet (2) and the stationary magnet (3) reaches the needle (5) by pushing. After the liquid drug is pushed, it enters through the inlet opening (8) on the stationary magnet (3) and reaches the needle (5) via the transfer channel (6). Said transfer channel (6) has a flexible and extensible structure in order to ensure the movement of the needle magnet (4) towards the exit opening (9).

There are locking elements (7) inside the injector body (1) to ensure that the piston magnet (2) and the needle magnet (4) remain fixed when the magnetic autoinjector is not in use. Said locking elements (7) lock the piston magnet (2) in the pulling direction, while locking the needle magnet (4) in the pushing direction. In this way, it is ensured that the liquid medicine is not sent to the needle (5) when the injector is not used, and that the needle (5) remains inside the injector body (1).

The working mode of the preferred embodiment of the invention is as follows:
Currently, the piston magnet (2) and the needle magnet (4) are locked with the locking elements (7) in the magnetic autoinjector. There is liquid medicine between the piston magnet (2) and the stationary magnet (3). When the liquid drug is desired to be injected into the living body, firstly the locking elements (7) are triggered and the piston magnet (2) and the needle magnet (4) are released. The released piston magnet (2) is attracted by the stationary magnet (3); the needle magnet (4) is pushed by the stationary magnet (3). During this movement, the liquid medicine between the piston magnet (2) and the stationary magnet (3) enters through the inlet opening (8) on the stationary magnet (3) and moves towards the needle (5) via the transfer channel (6). The needle magnet (4) also moves towards the exit opening (9) with the flexible structure of the transfer channel (6) and it is ensured that the needle (5) exits from the exit opening (9) and reaches the living body. As a result of this movement, the piston magnet (2) touches the stationary magnet (3), and the liquid medicine between them reaches the needle (5) through the transfer channel (6) and is injected into the living body.

After the injection process, the needle (5) is pressed against a hard place and the needle magnet (4) is brought to its initial position. In order to achieve this, the surfaces of the locking elements (7) facing the exit opening (9) are shaped obliquely. The needle magnet (4) opens the locking elements (7) by pushing their oblique surfaces, and when it passes to the upper side of the locking elements (7), locking is achieved again. In this way, it is ensured that the needle (5) stays in the injector body (1) so that it will not puncture again.

## Claims

1. A magnetic autoinjector that is used in cases where intradermal, subcutaneous or intramuscular injection is needed in the living body, allowing a certain dose of liquid medicine to be injected into the living body easily and safely, the magnetic autoinjector comprising:
• an injector body (1), which has a closed cylindrical form and has an outlet opening (9) at the end,
• a stationary magnet (3), positioned in a stationary manner in the injector body (1) mostly in the middle region of the injector body (1), with an inlet opening (8)
• a needle magnet (4) with a needle (5) on it that is positioned in the injector body (1) between the stationary magnet (3) and the outlet opening (9), being closer to the stationary magnet (3), being their same poles facing each other to enable them to apply a repulsive force,
• a piston magnet (2) located inside the injector body (1) on the other side of the stationary magnet (3), being their opposite poles facing each other in order to provide a pulling force to each other and with liquid medicine between them,
• a transfer channel (6), located inside the stationary magnet (3) and the needle magnet (4), which provides the transfer of the liquid medicine entering the inlet opening (8) to the needle (5),
• locking elements (7) that keep the piston magnet (2) and needle magnet (4) fixed against pulling and pushing forces when the injector is not in use.

2. The magnetic autoinjector according to claim 1, further comprising the needle magnet (4) containing a flexible or extensible transfer channel (6) that allows it to move away from the stationary magnet (3) to the outlet opening (9).

3. The magnetic autoinjector in accordance with Claim 1, further comprising locking elements (7) whose surfaces facing the outlet opening (9) are shaped obliquely, which ensures that when the needle (5) is punctured on a hard surface it remains inside the injector body (1) after the injection process.

## Patentansprüche

1. Magnetischer Autoinjektor, der in Fällen verwendet wird, in denen intradermale, subkutane oder intramuskuläre Injektion in den lebenden Körper erforderlich ist, die es ermöglicht, eine bestimmte Dosis flüssigen Arzneimittels einfach und sicher in den lebenden Körper zu injizieren, wobei der magnetische Autoinjektor Folgendes umfasst:
• einen Injektorkörper (1), der eine geschlossene zylindrische Form aufweist und an dem Ende eine Auslassöffnung (9) aufweist,
• einen stationären Magneten (3), der auf eine stationäre Weise in dem Injektorkörper (1), vorwiegend in dem mittleren Bereich des Injektorkörpers (1), positioniert ist, mit einer Einlassöffnung (8),
• einen Nadelmagneten (4) mit einer Nadel (5) darauf, der in dem Injektorkörper (1) zwischen dem stationären Magneten (3) und der Auslassöffnung (9) positioniert ist, wobei er näher an dem stationären Magneten (3) ist, wobei ihre gleichen Pole einander zugewandt sind, um es ihnen zu ermöglichen, eine abstoßende Kraft aufzubringen,
• einen Kolbenmagneten (2), der sich innerhalb des Injektorkörpers (1) auf der anderen Seite des stationären Magneten (3) befindet, wobei ihre entgegengesetzten Pole einander zugewandt sind, um eine Zugkraft zueinander bereitzustellen, und mit flüssigem Arzneimittel zwischen ihnen,
• einen Übertragungskanal (6), der sich innerhalb des stationären Magneten (3) und des Nadelmagneten (4) befindet, der die Übertragung des flüssigen Arzneimittels, das in die Einlassöffnung (8) eintritt, zu der Nadel (5) bereitstellt,
• Verriegelungselemente (7), die den Kolbenmagneten (2) und den Nadelmagneten (4) gegen Zug- und Druckkräfte fixiert halten, wenn der Injektor nicht verwendet wird.

2. Magnetischer Autoinjektor nach Anspruch 1, ferner umfassend den Nadelmagneten (4), der einen flexiblen oder erweiterbaren Übertragungskanal (6) enthält, der es ihm ermöglicht, sich von dem stationären Magneten (3) zu der Auslassöffnung (9) zu bewegen.

3. Magnetischer Autoinjektor nach Anspruch 1, ferner umfassend Verriegelungselemente (7), deren der Auslassöffnung (9) zugewandte Oberflächen schräg geformt sind, was sicherstellt, dass, wenn die Nadel (5) auf einer harten Oberfläche durchgestochen wird, sie nach dem Injektionsvorgang innerhalb des Injektorkörpers (1) bleibt.

## Revendications

1. Auto-injecteur magnétique qui est utilisé dans les cas où une injection intradermique, sous-cutanée ou intramusculaire est nécessaire dans le corps vivant, ce qui permet d'injecter facilement et en toute sécurité une certaine dose de médicament liquide dans le corps vivant, l'auto-injecteur magnétique comprenant :
• un corps d'injecteur (1), qui comporte une forme cylindrique fermée et comporte une ouverture de sortie (9) au niveau de l'extrémité,
• un aimant fixe (3), positionné de manière fixe dans le corps d'injecteur (1) principalement dans la zone médiane du corps d'injecteur (1), avec une ouverture d'entrée (8),
• un aimant à aiguille (4) sur lequel se trouve une aiguille (5) qui est positionné dans le corps d'injecteur (1) entre l'aimant fixe (3) et l'ouverture de sortie (9), plus proche de l'aimant fixe (3), leurs mêmes pôles se faisant face l'un l'autre pour leur permettre d'appliquer une force de répulsion,
• un aimant à piston (2) situé à l'intérieur du corps d'injecteur (1) de l'autre côté de l'aimant fixe (3), leurs pôles opposés se faisant face l'un l'autre afin de fournir une force de traction l'un vers l'autre et avec un médicament liquide entre eux,
• un canal de transfert (6), situé à l'intérieur de l'aimant fixe (3) et de l'aimant à aiguille (4), qui assure le transfert du médicament liquide entrant dans l'ouverture d'entrée (8) jusqu'à l'aiguille (5),
• des éléments de verrouillage (7) qui maintiennent l'aimant à piston (2) et l'aimant à aiguille (4) fixés contre les forces de traction et de poussée lorsque l'injecteur n'est pas utilisé.

2. Auto-injecteur magnétique selon la revendication 1, comprenant en outre l'aimant à aiguille (4) contenant un canal de transfert flexible ou extensible (6) qui lui permet de s'éloigner de l'aimant fixe (3) jusqu'à l'ouverture de sortie (9).

3. Auto-injecteur magnétique selon la revendication 1, comprenant en outre des éléments de verrouillage (7) dont les surfaces faisant face à l'ouverture de sortie (9) sont formées de manière oblique, ce qui garantit que lorsque l'aiguille (5) est piquée sur une surface dure, elle reste à l'intérieur du corps d'injecteur (1) après le processus d'injection.
